Europäisches Patentamt

European Patent Office

Office européen des brevets

(19)

(11) Publication number: **0 071 640**
**B1**

(12) **EUROPEAN PATENT SPECIFICATION**

(45) Date of publication of patent specification: **04.06.86**

(21) Application number: **82900887.9**

(22) Date of filing: **10.02.82**

(86) International application number:
**PCT/US82/00166**

(87) International publication number:
**WO 82/02774 19.08.82 Gazette 82/20**

(51) Int. Cl.⁴: **G 01 N 33/576, A 61 K 39/29**

(54) NON-A, NON-B HEPATITIS VIRUS.

(30) Priority: **11.02.81 US 233651**

(43) Date of publication of application:
**16.02.83 Bulletin 83/07**

(45) Publication of the grant of the patent:
**04.06.86 Bulletin 86/23**

(84) Designated Contracting States:
**FR**

(73) Proprietor: **CONNAUGHT LABORATORIES INCORPORATED**
**Swiftwater Pennsylvania 18370 (US)**

(72) Inventor: **MAUPAS, P.C.**
**deceased (FR)**
Inventor: **COURSAGET, Pierre L. J.**
**6 Place de Richemont**
**F-37170 Saint Avertin (FR)**

(74) Representative: **Kneissl, Richard, Dr.**
**Patentanwälte Andrae, Flach, Haug, Kneissl**
**Steinstrasse 44**
**D-8000 München 80 (DE)**

(56) References cited:
**WO-A-80/02598**
**WO-A-82/00205**
**US-A-4 196 265**
**US-A-4 217 145**
**US-E- 29 169**

**LANCET, 8217, 24 January 1981, SHU et al.,
"Specitifity of an immunoprecepitin test ...",
pages 178-180**

The file contains technical information
submitted after the application was filed and
not included in this specification

(56) References cited:
**LANCET, 8154, 15 December 1979, VITVITSKI
et al., "Detection of virus associated...", pages
1263-1267**

**LANCET, 8095, 21 October 1978, SHIRACHI et
al, "Hepatitis "C" antigen..."**

**LANCET, 8189, 9. August 1980, MORI et al.,
"Virus-like paticles...", page 318**

Courier Press, Leamington Spa, England.

**0 071 640**

(56) References cited:

SCIENCE, 205, 13 July 1979, SHIMIZU et al, "Non-A, Non-B hepatitis...", pages 197-200

JOURNAL OF GENERAL VIROLOGY, 48, 1980, NEURATH et al, "An antigen detected...", pages 285-295

CHEMICAL ABSTRACTS, vol. 92, no. 25, 23th June 1980, page 427, abstract no. 213068q, COLUMBUS OHIO (US).

## Description

This invention relates to the detection and prevention of infection by a newly discovered etiological agent for non-A, non-B (NANB) hepatitis. In particular this invention is concerned with making vaccines and conducting immunoassays for this agent or its antibodies.

NANB hepatitis is defined as clinical hepatitis which cannot be attributed to infection by cytomegalovirus, Epstein-Barr virus, or hepatitis A or B.

NANB hepatitis was first identified as transfused individuals. Transmission from man to chimpanzee and serial passages in chimpanzees provided evidence that NANB hepatitis is due to an infectious agent or agents. NANB hepatitis represents up to 90% of post-transfusion hepatitis cases since hepatitis B antigen—positive blood is no longer used for transfusion. The risk of contracting NANB hepatitis after blood transfusion has been estimated to be close to 10% in the U.S. Therefore, it is important to screen the blood of potential donors to detect NANB hepatitis. Further, use of donors vaccinated against NANB hepatitis would serve to reduce the probability of transmitting serum hepatitis.

NANB hepatitis is associated with a variety of virus-like particles found in serum and tissue extracts. Hollinger et al., in "Proc. Second Symposium on Viral Hepatitis" (San Francisco), p. 699, (1978) report the previous work of others in locating 20—22 nm and 60—80 nm particles associated with NANB hepatitis. Other particles which have been reported to be associated with NANB hepatitis are (a) small spheres and filaments 15—25 nm in diameter, (b) 35—40 nm diameter virions which resemble the hepatitis B DANE particle, (c) a 27 nm virus-like particle identified in lots of antihemophilic factor and liver tissue from chimpanzees infected after administration of antihemophilic factor ("Morbidity and Mortality Weekly Report" 27 (21) [1978]), and (d) a 20—27 nm diameter particle identified in hepatocytes of infected animals (Shimizu et al., "Science" 205: 197—200 [1979]). In addition, antigen-antibody systems not linked to a definitive particle have been identified as associated with NANB hepatitis (Kabiri et al., "Lancet", August 4, 1979, pp. 221—224; Vitvitski et al., "Lancet", December 15, 1979, pp. 1263—1267; Dienstag et al., "Lancet", June 16, 1979, pp. 1265—1267; and Shirachi et al., "Lancet" October 21, 1978, pp. 853—856.

Objects of the invention

A principal object of this invention is to locate and isolate virus particles which are etiological agents for NANB hepatitis.

An additional object is to purify the particles without altering their antigenic characteristics.

A further object is to prepare vaccines from the particles.

Another object is to elicit or harvest antibodies against the particles for administration as therapeutic gamma globulin or for use in immunoassays for detecting the particles.

A further object is to produce water insoluble particles and antigens thereof, and insolubilized antibodies capable of binding the particles antigens, for use in immunoassays or for affinity chromatographic purification of the particle antigens or antibodies.

A still further object is to provide labelled, particularly enzyme or radiolabelled, particle antigens or antibodies.

These and other objects of the invention will be apparent from consideration of this specification as a whole.

Summary of the invention

The inventors have discovered two unique viral particles which are associated with NANB hepatitis. These particles are principally differentiated on the basis of their size, although other characteristics are helpful in classifying them.

The first particle resembles a togavirus. It is a spherical, coated virus having an outer diameter of 50—60 nm and a core having a diameter of about 40 nm. Heating an aqueous suspension of this virus at 25°C for extensive periods or exposing the virus to ether causes it to lose its infectivity for tissue culture. It has been recovered from the plasma or urine of patients suffering from NANB hepatitis. Four samples have been placed on deposit at the Atlantic Type Culture Collection (ATCC): VR-2011, VR-2012, VR-2013 and VR-2014. This virus forms no part of this invention but rather is described and claimed in European patent application 81901986.0. The second particle resembles an enterovirus and forms the basis of the present invention. This enterovirus like particle is spherical, having a diameter of about 27 nm; exhibits a buoyant density of 1.36 in isopycnic density-gradient centrifugation with cesium chloride; loses its tissue culture infectivity upon heating in aqueous suspension for 3 hours at 98°C, but not after 10 hours at 56°C; it is insensitive to ether; and its genetic material is positive to a histochemical stain for RNA. A sample has been deposited with ATCC on July 1, 1980 and has been given accession No. VR-2015.

The objects of this invention are achieved in part by novel compositions made possible by the discovery of the above deposited virus particles as well as the parallel discovery that both may be cultured in vitro. The compositions comprise

(a) the purified virus particles or their antigens, particularly those substantially free of primate protein such as human serum protein;

(b) the viral particles rendered biologically noninfectious or their antigens for use as a vaccine;

(c) primate immunoglobulin which is substantially free of other primate proteins and which is capable of binding at least one antigen of the virus particles;

(d) nonprimate antibody which is capable of binding an antigen of the virus particles;

(e) the particles and their antigens or antibodies made water insoluble substance for use in immunoassays or chromatographic separations; and

(f) the particles and their antigens or antibodies bound to a detectable label for use in immunoassays.

Further, the above objects are achieved by providing methods for making the compositions; immunoassays comprising detecting or determining an antigen of the viral particle; immunoassays comprising detecting or determining IgM or IgG capable of binding antigen of the viral particle; and a method for propagating ATCC VR-2015 comprising culturing it in a diploid cell culture.


Detailed description of the invention

Unless further qualified, the term "particle" henceforth refers to the enterovirus-like particle ATCC VR-2015. "Particle" is not intended to describe the physical state of the virions.

A particle antigen is defined as a substance, normally a protein, containing at least one epitopic site of the deposited particle. Thus, "particle antigen" includes antigens which are present in particles otherwise immunologically distinct from the particle as deposited. A particle antigen may also be present on a viral fragment or in solution, in both cases being free of any other antigenic sites otherwise present on the complete deposited particle.

Particle antibody is defined as an antibody capable of binding a particle antigen, regardless of the origin of the antibody. Such antibody may be obtained from convalescent serum or by active immunization of humans or lower animals with particle antigen as is more fully described below. Also, either particle antibody or antigen may be purified or in association with other substances, e.g. stabilizers, carriers and serum, unless such substances are specifically excluded.

The enterovirus-like particle ATCC VR-2015 was obtained from the stool of an acute phase NANB hepatitis patient. A chimpanzee inoculated with an extract of feces of an acute hepatitis patient developed a NANB biological hepatitis. The particle was observed later in samples of the chimpanzee's liver. The particles* were found to clump upon mixture with convalescent sera from 4 out of 14 NANB hepatitis patients tested, as visualized by immune electron microscopy. Thus these two particles are implicated in a significant proportion of NANB hepatitis cases.

The particle may be cultured *in vitro* in tissue culture. Surprisingly, the enterovirus-like particle is particularly prolific in different diploid cell types and culture media. While tissue culture is a

---

*A similar enterovirus-like particle was observed in the stool of an acute phase NANB patient.

well-known and conventional art for growing many viruses, hepatitis-associated viruses have proven to be generally difficult to culture *in vitro*. Although earlier reports to the contrary do exist, Cossart (*Virus Hepatitis and its Control*, Bailliére Tindall, London, p. 40) observed in 1977 that the viral agent for hepatitis B had eluded all efforts to cultivate it *in vitro*. Applicants' method for culturing the particle has facilitated the manufacture of vaccines and immunoassays as will be further described below.

The principal variables in the tissue culture method of this invention are cell source, culture medium, temperature and the timing for inoculation and virus harvest. Unless described below to the contrary, none of these variables is critical within the limits one skilled in the art would ordinarily place in general on the tissue culture of a virus. Selection and optimization of each parameter will be within the skill of the ordinary artisan even though some routine experimentation will be required. It is important to note that applicants' principal contribution lies not so much in the selection of conditions and reagents as in the discovery that the heretofore unknown particle can be cultured *in vitro* at all.

The cells used in the culture must be diploid cells, but otherwise the source is not significant. Fresh cultures of cells prepared in conventional fashion from adult or fetal organs, e.g. lung, kidney or liver, may be used, but it is more convenient and manufacturing is better controlled by cell cultures stable to indefinite replication, i.e. cell lines. Representative examples of suitable cell lines are MRC-5 and WI-38, all of which are available from depositories. The cells are preferably primate although other animal cells suffice.

Any culture medium conventionally used to grow and maintain the susceptible cell line is satisfactory for initially culturing and then sustaining the cells during viral replication. The preferred medium is disclosed by Morgan et al., "Proc. Soc. Biol. Med." 73:1 (1950). It is supplemented with antibiotics and a small proportion, usually about from 5% to 15% by volume, of human or animal serum. If human serum is used it should obviously be free of antibodies to the particles. Air is a suitable atmosphere; the culture container should be ventilated in a sterile fashion.

The cultivation temperature is lower than the biological inactivation temperature of the particle in tissue culture, readily determined by cultivating the particle over a range of temperatures. The enterovirus-like particle is considerably stable. Generally, this particle may be cultured through a large number of passages at up to about 40°C, ordinarily about from 30—40°C. Optimal cultivation temperatures will vary depending primarily upon the cell source and growth medium composition.

The tissue culture is allowed to grow to confluence on the surfaces of a conventional culture chamber, so as to maximize the population of susceptible cells. Innoculation with particle should then follow promptly, especially if a stable cell line is not being used. An inoculum containing about from $1\times10^5$ to $1\times10^8$ particles/ml may be used, ordinarily from prior culture although pathological samples may be used so long as they are pretreated to remove substances which are toxic to the cell culture. It is not necessary to exchange the tissue culture medium for brief cultivation periods. However, waste product buildup can interfere with cell survival and viral replication after about 2 days, so a medium exchange may be desirable.

The culture should continue for about from 1 to 7 days to achieve maximum particle yields. This period will vary considerably and should be determined for each set of conditions.

Yields of the enterovirus-like particle are high, on the order of $1\times10^9$ particles/ml. These particles are infective for further tissue culture, and have remained so for over 20 passages. The particles are recovered from the culture by simply decanting and combining the spent media from the culture container and then purifying them using one or more of the procedures described below. The purified particles are preferably frozen in a medium consisting of 50% of the growth medium without serum and 50% glycerol by volume.

The particles may also be reproduced *in vivo* by infecting a chimpanzee with pathological material. The animal is monitored for hepatitis symptoms. Plasmapheresis can then be used to recover large quantities of infected plasma during the acute phase. Antibody can also be secured from this plasma. Alternatively, the animal may be sacrificed and the liver excised and homogenized as a source of particles.

It may be desirable for a variety of reasons to purify the particles present in pathological material such as tissues, urine and blood fractions, or which are obtained through tissue culture. For example, if the particle is to be treated and employed as a vaccine or in an immunoassay, there ordinarily should be as little in the way of extraneous protein contamination as possible. Thus, the particle or its antigens should be substantially free of primate proteins. The term substantially free does not mean that the particle antigen is devoid of proteins present in its natural environment which are noncovalently bound or adsorbed thereto, e.g. antibodies. However, the term does mean that all but trace concentrations of the unbound proteins normally associated with the particle or particle antigen in the pathological material or tissue culture supernatant have been removed, e.g., normal human serum proteins, urinary albumin and cellular proteins.

Particle antigens are purified by first being separated from water insoluble contaminants having greater dimensions or different densities. Such contaminants may include animal cell debris, e.g., from tissue culture; cellular microorganism, e.g., bacteria inurine; or chylomicra in sera. This gross separation is generally accomplished by low speed centrifugation or filtration, the parameters of which will obviously vary depending upon the nature and degree of contamination and can be determined by routine experimentation. Filtration is preferred as an initial step in processing large volumes of particle suspension. Ordinarily, filters having an average pore diameter of 0.8 μm may be used to retain gross contamination and pass the particles.

The antigens may be separated from undesired materials after gross contamination is removed. Suitable techniques include precipitation, ultrafiltration and ultracentrifugation, all of which are capable of distinguishing particles and particle fragments or antigens from relatively lower molecular weight, water soluble contaminants such as proteins and salts. For example, a gross filtrate such as described above may be ultrafiltered with a membrane having a molecular weight cut-off intermediate the approximately 50 million of the particle and the nearly 1 million of most commonly encountered protein contaminants. The retentate is washed with a buffered solution, the washed retentate recovered and then mixed with buffers and stabilizers such as hydroxyl compounds, e.g., sugars, glycerol or carbohydrates; sulfhydryl compounds such as cysteine; and proteins such as serum albumin.

It may be more convenient to precipitate the particles or their large molecular weight fragments using one or more various well-known flocculating or protein precipitating agents, particularly salting out agents or flocculating polymers. Examples include polyethylene glycol and ammonium sulfate. The precipitating agent may be readily removed from the precipitate by dialysis and the particles resuspended in buffered stabilizer solutions for further purification or use.

The concentration of polyethylene glycol to be used will depend upon which particle is to be purified, whether or not the particles are intact, the electrolyte constituents of the sample, the temperature, the molecular weight of the polyethylene glycol and the particle concentration. In general, polyethylene glycol of average molecular 6000 may be added to serum to a concentration of about from 10 to 20% by weight at a temperature of about from 1° to 5°C to precipitate the particles. Precipitation from urine may not require as much polyethylene glycol as with serum.

The ammonium sulfate concentration to be used is dependent upon the factors noted above for polyethylene glycol, except that the molecular weight of the precipitant is obviously not a variable. Generally, ammonium sulfate at about from 12 to 18% of saturation is satisfactory to precipitate the particles.

Continuous ultracentrifugation is preferred for making vaccines as it is a physical process that introduces no potential contaminants and can be practiced economically in large scale. Other methods which can be used to purify the particles or their antigens include centrifugal sedimentation in a sucrose or cesium chloride gradient, zone electrophoresis, or ion exchange, gel or affinity chromatography.

Chromatography is a versatile method since it may be readily scaled up for commercial manufacture of the particles. Gel chromatographic systems using cross-linked dextran beads are preferred due to the comparatively low cost. A column of suitable gel can be selected which will permit diffusion of proteins and low molecular weight substances into the void volume of the gel beads, thereby retarding the progress of these contaminants through the column, while allowing the particles to pass through virtually unimpeded. The gel which is selected will thus be a matter of routine experimentation.

Any of the above methods may be combined as desired. For example, gel chromatography of contaminated serum on Biogel A5M and subsequent isopycnic centrifugation in cesium chloride is effective in removing human serum proteins.

If a large proportion of the particle antigens present in a sample are particle fragments or water soluble protein antigens, then the most expeditious technique for separating them may be affinity chromatography. Antibodies capable of binding the particle antigens are covalently linked or absorbed to an insoluble support using conventional procedures. The insoluble antibody is placed in a column and the sample is passed through. Immunologically-bound antigen is washed with buffer and then released by changing the ionic strength or pH of the wash buffer. Generally, acid pH is effective at releasing the bound antigen. Antigen prepared in this fashion is essentially free of contaminant proteins.

The contaminating substances which may be removed from pathological samples include cellular microorganisms, urea, bilirubin, uric acid, albumin, mucins, electrolytes such as calcium, iron, sodium, potassium, magnesium, chloride, phosphate, ammonia and sulfate, cholesterol, fatty acids, proteins such as albumin, $\alpha_1$-globulins, $\alpha_2$-globulins, B-globulins, fibrinogen and lipoproteins.

The particles obtained from pathological sources such as serum or urine may be immunologically complexed with antibody, but antigens essentially free of adherent antibody are desirable for use as reagents in immunoassays.

Such purified particles or their antigens may be made free of primate proteins, including any particle-bound antibodies, by tissue culture as described above but in a culture medium free of undesired proteins, e.g. cells growing in a culture medium containing no human protein. Even if the original pathological sample is primate, after several passages through tissue culture the initial contaminants are diluted to undetectable concentrations.

Purified antibody to particle antigen is also useful in immunoassays. The impure antiserum source may be the sera of patients convalescing from a NANB hepatitis infection or an animal which has been immunized against the particle antigens. The immunized animals may be non-primates, generally guinea pigs, rabbits, goats or horses, which are not susceptible to hepatitis and thus can be injected with particles which would otherwise be infective and potentially lethal. The antibody-containing gamma globulin fraction may be purified by any conventional protein fractionation procedures previously employed to separate gamma globulin from plasma, e.g., alcohol, polyethylene glycol or ammonium sulfate precipitation.

The degree of antibody purity desired will depend upon the source and intended use. Gamma globulin for therapeutic or prophylactic administration in the treatment or prevention of NANB hepatitis should be of human origin and should be of as high degree of purity as commercially feasible. Usually, this at least entails removing the other desirable fractions of human plasma, e.g., antihemophilic factor or prothrombin complex, from the donor plasma.

Vaccines can be made from the enterovirus-like particle, fragments and water soluble antigens thereof. Intact particles are preferred because they necessarily comprise the complete spectrum of antigenic determinants and will confer the highest degree of immunity. It is also desirable that the vaccines be free of non-human proteins from such sources as the nutrient medium, cell lines or tissues in which the virus is cultured. Intact particles meeting this requirement may be made by culturing the viruses on human cells in a medium which contains only human protein or a protein substitute such as synthetic, low molecular weight peptides.

Intact particles for use as vaccines must be attenuated or inactivated to eliminate biological infectivity. This may be accomplished by treatment with an agent such as formalin that will not significantly affect the immunogenic or antigenic characteristics of the preparation. A satisfactory procedure entails adding formalin to the particle-containing solution or suspension and incubating under conditions which will destroy the infectivity of the composition, usually incubation at about from 25° to 40°C for about 1 to 5 days, or by heating under the inactivation conditions for the two particles as described above. Formalin is removed or neutralized after inactivation, although a bacteriostatically effective amount of formalin, e.g., 10 ug/ml, may be left in the vaccine. The viral particle count is adjusted to about from $1 \times 10^7$ to $1 \times 10^{11}$ by the addition of a physiologically acceptable carrier such as saline, by removal of excess diluent through ultrafiltration, or by lyophilization and reconstitution to the desired concentration. An adjuvant may also be added.

Vaccination may be conducted in conventional fashion, for example by subcutaneous administration of 1 ml amounts of the vaccine at one month intervals for a total of three immunizations. A booster at one year may be required.

Any of the immunoassay methods heretofore disclosed in the art may be used to determine particle antigens or antibodies. The following disclosure is generally expressed in terms of determining antigens. However, antibodies may be determined using the same methods.

The analytical methods described herein for assaying particle antigens or antibodies should not be construed as requiring the determination of all of the particle antigens or epitopic sites. Similarly, it is not necessary to assay for all of the antibodies which can bind the population of epitopic sites presented by the particle. The reason for this is that mutations and varieties of viruses are frequently found in nature. Thus, some antibodies originally obtained by immunizing an animal against particles may not bind, or may bind very weakly to related NANB hepatitis strains. Fortunately, the viral particle described herein contains so many epitopic sites that the failure of some antibodies to bind variant sites will not significantly affect immunoassay effectiveness; a sufficient proportion of the antibody population will remain capable of binding antigens common to both the immunizing and test viruses. It is even possible to base the immunoassay upon two or, in some cases, one epitopic site. The antibody used in detecting particle antigens may be raised by immunization against applicants' deposited particles, against strains newly isolated from nature containing cross-reacting antigens, or against a fragment of those strains or new isolate. Similarly, particle antigens used in immunoassays for antibody may be derived from the deposited particles, a new isolate of a virus containing a cross-reacting antigen or a fragment of either the deposited particles or new isolate. A cross-reacting particle antigen is one which binds an antibody produced against an antigen of another virus with at least about 40% of the avidity as the antibody binds the antigen of the immunizing virus.

The antibodies will generally fall into two diagnostically interesting classes, immune globulins G (IgG) and M (IgM). It is preferred to assay IgM as it is the antibody class which first appears during the course of infection, when IgG synthesis may not yet have been initiated. However, IgG assays may be material for governmental regulation because this particular fraction is detectable for many months after infection, thus demonstrating that a potential blood donor has at one time been exposed to the particle antigen even though the donor may no longer be infectious.

Many suitable methods for determining IgG or IgM exist. For example, the IgG and IgM fractions of a test sample may first be separated by conventional procedures, most conveniently by specifically absorbing either fraction to an insoluble surface. For example, insoluble anti-IgG or anti-IgM antibodies may be produced in a first animal species against the IgG or IgM of another animal species. Alternatively, Boyle et al., ("J. Immunological Methods" 32 (1):51—8 [1980]) disclose that immobilized staphylococcal Protein A and concanavalin A will bind IgG or IgM antibodies, respectively. Thus, any of the foregoing specific adsorbents may be employed to separate IgG from IgM. Then a conventional immunoassay may be used to determine the separated globulin fraction.

Either heterogeneous or homogeneous immunoassay techniques are satisfactory for determining particle antigens or antibodies. Heterogeneous assays are preferred in determining intact particles or large antigenic fragments. Heterogeneous methods all include a step of separating a solid phase with bound analyte from an aqueous phase. In such assays an antigen or antibody is first insolubilized, as for example by adsorption onto plastic beads or the inner wall of a plastic test tube, by covalent linkage to an insoluble substance or by binding to a specific binding partner already so adsorbed by linking.

One heterogeneous assay is the "competitive" method. Here, unlabelled sample analyte, e.g. antigen or antibody, and labelled analyte analogue compete for a limited number of insoluble sites capable of binding the analyte. Then the insoluble, bound analyte is separated from the free analyte and the distribution of labelled analyte determined. This method is preferred for the assay of particle antibodies. In such a case, the particle antigen is insolubilized, and labelled and sample particle antibody compete for limited antigen sites.

The "sandwich" heterogeneous method is also suitable for determining particle antigen or antibody, preferably for determining particle antigen as intact particles or large fragments. The method comprises adsorbing the sample analyte onto insoluble binding partner, removing the residual sample, adsorbing labelled binding partner onto the now insoluble analyte, removing excess labelled binding partner and determining the distribution of label, usually by assaying the amount of label bound to the solid phase. The insoluble binding partner is preferably particle antibody. A variant of this method comprises directly adsorbing antigen from the sample onto a nonspecific adsorbent, e.g., a polyolefin, followed by labelled particle antibody or, sequentially, particle antibody and labelled antigen.

Another suitable technique is sequential saturation. As with the sandwich method, a surplus of insoluble binding partner to analyte is used to bind the sample analyte, if any. However, rather than next contacting the insoluble analyte with labelled binding partner, labelled analyte analogue is added to occupy the remaining insoluble binding sites. Unbound labelled analyte is removed and the label content of one of the separated phases is determined.

Homogeneous immunoassay methods may also be employed to advantage. These methods have in common the elimination of the above-described phase separation; the complete determination can be conducted entirely with water soluble reagents. One such method for determining particle antigen comprises labelling particle antigen with an enzyme to form a conjugate, following by admixing the conjugate, sample and antibody to the antigen, and finally determining the change in enzyme activity brought on by binding of the conjugate with antibody not occupied by antigen in the sample (U.S. Patent 3,817,837, incorporated herein by reference).

Another homogeneous assay, which may also be used in a heterogeneous mode, is disclosed in U.S. Patent 3,935,074, also incorporated by reference. In an embodiment of this method for determining an antigen, a conjugate is formed by covalently linking the antigen with a detector ligand. Antibodies to both the antigen and detector ligand are then mixed with the conjugate and sample. Then the usual practice is to measure the residual, unbound antibody to the detector ligand; it will be found in inverse relation to the amount of particle antigen.

Other suitable assay systems are disclosed in U.S. Patents 4,006,360; 4,134,792 and 3,996,345; Yorde et al., "Clinical Chemistry" 22 (8): 1372—1377 (1976) and U.S. Patent application serial number 572,008.

The labels to be used in the above methods are well-known. Examples include radioisotopes, enzymes, coenzymes, enzyme modulators, stable free radicals, and fluorescent and luminescent groups. Preferred labels are radioisotopes, particularly $^{125}$I, or enzymes.

The particle antigens or antibodies may be labelled with radio-iodine in any conventional manner. Suitable methods use chloramine-Tor lactoperoxidase, e.g., as disclosed by Dermody et al., "Clinical Chemistry" 25 (6):989—995 (1979 or Parsons et al., "Analytical Biochemistry" 95:568—574 (1979, externally radio-iodinated small molecules such as ($^{125}$I) iodohydroxyphenyl propionate - N - hydroxysuccinimate ester (Bolton et al., "Biochem. Journal" 133:529—533 [1973]), ($^{125}$I) diiodofluorescein isothiocyanate (Gabel et al., "Analytical Biochemistry" 86:396—406 [1973]), tertiary - butoxycarbonyl - L - ($^{125}$I) iodotyrosine - N - hydroxysuccinimide ester (Assoian et al., "Analytical Biochemistry" 103:70—76 [1980]), or IC (Montelaro et al., "Analytical Biochemistry" 99:92—96 [1979]). The technique disclosed by Montelaro et al. is preferred for labelling the surface of ATCC VR-2015.

The invention will be more fully understood by reference to the following examples.

Example 1

The enterovirus-like particle ATCC VR-2015 may be radioiodinated by the following method. It is suspended in phosphate buffered saline at pH 7.4. A stock solution of ICl is prepared by diluting 0.56 g KI, 0.33 mg $NaIO_3$, 29.2 g NaCl and 210 ml of concentrated HCl to 250 ml with distilled water. Immediately before use, this stock solution is extracted several times for 2 min. with 3 ml of chloroform until the chloroform is no longer colored pink. Residual chloroform is removed from the ICl stock by bubbling water saturated air for 5 min. Finally, 1 ml of ICl stock is thoroughly mixed with 10 ml of 2M NaCl. 0.1 ml of particle suspension is diluted with an equal volume of 1M glycine buffer (pH 8.5) and mixed with 1 mCi of Na[125]I in 0.1 ml of the final ICl preparation and allowed to proceed for 5—10 seconds. The product is rapidly separated from unreacted $^{125}$I by gel filtration through a column (0.9×15 cm) of Sephadex G-25 beads and eluted with phosphate buffered saline.

Example 2

A vaccine may be made by adding formaldehyde to a purified suspension of the enterovirus-like particle ($1×10^9$/ml in phosphate buffered saline) to a final concentration of 1:4000 and the mixture incubated with continuing agitation for 1 day at 37°C and then 5 days at 4°C. The reaction mixture is partially neutralized with sodium bisulfite to leave a final concentration of 10 ug/ml formaldehyde. The vaccine is stored at 4°C.

The vaccine is subcutaneously administered to experimental animals for 14 weeks to demonstrate the potency of the vaccine.

Example 3

For culture of ATCC VR-2015, the culture medium of Morgan et al., ("Proc. Soc. Exp. Biol. Med.", 73:1 [1950]) is modified by adding 10% foal serum, 50 ug of dihydrostreptomycin/ml and 100 units of benzylpenicillin/ml. The medium is sterilized by filtration through a 0.22 micron filter. An aliquot of the medium is placed into a plastic Falcon flask, followed by an inoculum of WI38 human diploid cells. The inoculated medium is incubated at 37°C until cellular confluence is achieved. The inoculum is about $1×10^9$ particles/ml of ATCC VR-2015 in a solution containing one half by volume of the culture medium without foal serum and one half part glycerol. The cell culture is a monolayer of MRC-5 diploid cells. When the population of virus particles reaches about $1×10^9$/ml in the supernatant culture medium, accompanied by cytotoxic changes in the cell monolayer, the culture is decanted, centrifuged at 1500×g to remove cell debris and harvested by continuous ultracentrifugation. The population of recovered particles was adjusted to about $1×10^{11}$ particles/ml by dilution into a suspending medium consisting of one half part by volume of the culture medium without foal serum and one half part glycerol.

Example 4

The purified ATCC VR-2015 recovered in Example 3 is emulsified in an equal volume of Freund's complete adjuvant in accordance with known procedures. The antigen is then injected into the peritoneum of a rabbit, followed by an intramuscular booster at 6 weeks. After 14 weeks, serum is harvested from the animal and the globulins precipitated with 40% ammonium sulfate. The precipitate is dissolved and dialyzed overnight against 200 volumes of 0.04 M phosphate buffer (pH 7.2).

## Example 5

50 ug aliquots of the antibody fraction of Example 4 are radiolabeled with $^{125}$I following Greenwood et al., "Biochem. J." *89*:114—123 (1963). The labeled IgG is separated from free radioiodine by passage through a column of Sephadex G-50®. The labelled IgG is diluted to about 3 uCi/ml in a diluent of 50% calf serum, 5% human serum negative for antibody to particle antigen and 0.1% $NaN_3$. This preparation is stored at 4°C.

## Example 6

The antibody to the enterovirus-like particle is purified by affinity chromatography. The purified particle obtained in Example 3 is bound to charcoal as follows: prewashed, sorbent carbon is packed into a column. The particle suspension is introduced into the column at a flow rate of approximately 200 ml per hour and recirculated until the column is saturated, i.e., until no further decreases in particle titer occur upon passage through the column. Residual nonspecific binding sites are saturated by passing normal rabbit serum through the column, followed by washing with 0.04 M phosphate buffer, pH 7.2 (PBS). Rabbit serum is chosen because a rabbit is the animal immunized with the enterovirus-like particle in Example 4. The collected rabbit serum is diluted into PBS 1:2 and added to the immunoadsorbent column at a flow rate of about 100 ml per hour. The column effluent is assayed for particle antibody in accordance with the method of Example 9. Serum input is stopped when particle-specific antibody begins to appear in the effluent. The column is washed with PBS until the minimum effluent protein content is reached as measured by ultraviolet adsorption. A 5 M NaI solution is passed through the column at about 100 ml per hour to elute the antibody from the particles. The collected effluent is ultrafiltered using an Amicon casette and 0.01 M sodium phosphate to remove NaI and concentrate the solution. The solution is then filtered through a 0.22 μm membrane and stored and frozen at −70°C until used.

## Example 7

The unlabelled antibody solution prepared in Example 6 may be labelled with an enzyme. Many suitable, well-known methods exist for accomplishing such labelling. The following method, based on U.S. Patent Re. 29,169 is satisfactory:

5 mg of antibody and 20 mg of horseradish peroxidase (HRP) are dissolved in 2 ml of 0.05 M phosphate buffer of pH 6.2. 40 μl of 25% glutaraldehyde is added and the solution shaken at room temperature for 2 hours. Then the mixture is dialyzed against PBS containing 0.001 Mg++ to remove glutaraldehyde, centrifuged at 250 xg, diluted in 0.05 M tris (hydroxymethyl) aminomethane buffer (pH 8), 1% normal human albumin 0.02% $NaN_3$ and 0.001 M $MgCl_2$. The residual unlinked enzyme may be removed by gel chromatography in conventional fashion.

Other enzymes which may be coupled to the antibody include dehydrogenases such as alcohol, glycerol, L-lactate, malate, glucose 6-phosphate and mannitol 1-phosphate dehydrogenase; oxidases such as glucose, galactose, L- or D-amino acid, polyphenol or ascorbate oxidase; catalase; cholinestease; alkaline phosphatase; B-glucoronidase; aldolase; and histidase. Considerations in selecting the enzyme include cost, capacity to be bound to the antibody, enzyme assay convenience, enzyme stability and absence of the enzyme in the biological fluids to be assayed.

## Example 8

1 ml of unfractionated guinea pig serum obtained in substantially the same fashion as described in Example 4 with respect to rabbits is coated onto the inner surface of a polypropylene test tube in accordance with U.S. Patent 3,646,346. 0.1 ml of a urine specimen from an acute NANB hepatitis patient and 0.1 ml of a control specimen know to be free of the particles are then placed into coated tubes and incubated for 12 hours. The unbound elements of the sample are washed from each tube with water, labelled antibody of Examples 5 or 7 added to each tube and incubated for 12 hours, unbound labelled antibody washed from the tube with water and the radioactivity or peroxidase activity bound to the tube determined. The particle antigens could be satisfactorily detected by determining the extent of bound radioactivity or peroxidase activity in the assay tube compared to the control tube. The procedure of this Example is effective for either particle and for serum rather than urine samples.

## Example 9

This method is representative of techniques for assaying anti-viral antibodies. Solutions of rabbit antihuman immune globulin were prepared at 1:1000 in 0.01 M tris buffer, pH 9.0. Following the procedure of U.S. Patent 3,646,346, 1 ml aliquots of the solution are placed into small, disposable polypropylene test tubes and allowed to coat overnight. The tubes were then washed and air-dried.

A serum sample and negative control is diluted 1,000 fold into 50% calf serum, 0.2% Tween-20 and 0.005 M EDTA in PBS. Duplicate 0.2 ml aliquots of the test samples are incubated overnight in the coated test tubes and then washed twice with 5 ml of water. 0.2 ml of the radiolabelled enterovirus-like particle made in accordance with Example 1 is added to each tube and incubated overnight. The label is decanted and the tubes are washed twice with 5 ml of water. The tubes are then counted in a gamma counter. The averaged count rates are compared with the negative control to determine whether or not the test sample is antibody positive.

If the test tube is initially coated with rabbit anti-human immune globulin which is specific for human IgG (prepared in known fashion), then the assay will be directed only to the gamma globulin portion of the test sample which is specific for the particle. Similarly, rabbit anti-IgM can be used to produce an assay specific for anti-particle IgM.

**Claims**

1. A diagnostic method for the detection of a newly discovered etiological agent for NANB hepatitis, which comprises assaying a biological sample for the antigen of viral particles having a diameter of about 27 nm, exhibit a buoyant density of 1.36 in isopycnic density-gradient centrifugation with cesium chloride, lose their tissue culture infectivity upon heating in aqueous suspension for 3 h at 98°C but not after 10 h at 56°C, being insensitive to ether and having genetic material positive to histochemical stain for RNA, which particles have ATCC No. VR-2015.

2. A diagnostic method for the detection of a newly discovered etiological agent for NANB hepatitis, which comprises assaying a biological sample for antibody to viral particles having a diameter of about 27 nm, exhibit a buoyant density of 1.36 in isopycnic density-gradient centrifugation with cesium chloride, lose their tissue culture infectivity upon heating in aqueous suspension for 3 h at 98°C but not after 10 h at 56°C, being insensitive to ether and having genetic material positive to histochemical stain for RNA, which particles have ATCC No. VR-2015.

3. The method of claim 2 wherein the antibody is IgG.

4. The method of claim 2 wherein the antibody is IgM.

5. A method which comprises culturing ATCC VR-2015 in vitro in cell culture.

6. The method of claim 5 wherein the culture is WI-38 or MRC-5 diploid cells.

7. The method of claim 1 wherein the sample is assayed for said antigen by contacting the sample with water insoluble antibody to said antigen, removing the sample, contacting the insoluble antibody with labelled antibody to said antigen, removing unbound labelled antibody from the insoluble antibody and assaying the label in either the bound or unbound fractions.

8. The method of claim 2 wherein the sample is assayed for antibody by contacting the sample with water insoluble specific binding partner for the test sample antibody, contacting the insoluble antibody with a labelled ATCC VR-2015 antigen, removing the unbound specific binding partner from the insoluble antibody and assaying the label in either the bound or unbound fractions.

9. The method of claim 8 wherein the specific binding partner is staphylococcal protein A or an antibody.

10. The method of claim 9 wherein the binding partner antibody is specific for IgG or IgM.

11. The method of claim 2 wherein the sample is assayed for said antibody by contacting the sample with water insoluble ATCC VR-2015 antigen and labelled antibody to said antigen, removing said sample and unbound labelled antibody from the insoluble antigen, and assaying the label in either the bound or unbound fractions.

12. A composition comprising an antigen of ATCC VR-2015 which is substantially free of primate proteins.

13. The composition of claim 12 wherein the antigen is a component of an intact ATCC VR-2015 particle.

14. A sterile composition comprising ATCC VR-2015 antigen in a pharmaceutically acceptable carrier.

15. The composition of claim 14 wherein the antigen is present as a component of biologically noninfectious ATCC VR-2015 particles.

16. The composition of claims 14 or 15 which is dry.

17. A composition comprising primate immunoglobulin which is substantially free of other primate proteins and contains antibody for binding an antigen of ATCC VR-2015.

18. A composition comprising nonprimate antibody for binding an antigen of ATCC VR-2015.

19. A composition comprising ATCC VR-2015 antigen bound to a detectable label or a water insoluble carrier.

20. The composition of claim 19 wherein the label is an enzyme, radioisotope, fluorescent group, luminescent group, coenzyme, enzyme modulator or stable free radical.

21. A composition comprising antibody to ATCC VR-2015 antigen which is bound to a detectable label or a water insoluble carrier.

22. The composition of claim 19 wherein the carrier is a polyolefin surface to which the antigen is adsorbed.

23. The composition of claim 21 wherein the carrier is a polyolefin surface to which the antibody is adsorbed.

24. A composition comprising sterile gamma globulin to ATCC VR-2015 antigen, which globulin is essentially free of antihemophilic factor and prothrombin complex.

25. The composition of claim 24 which is dry.

**Patentansprüche**

1. Diagnostisches Verfahren zum Nachweis eines neuentdeckten ätiologischen Wirkstoffs der NANB-Hepatitis, das die Untersuchung einer biologischen Probe auf das Antigen von Virusteilchen mit einem Durchmesser von etwa 27 nm umfaßt, die eine Schwebedichte von 1,36 bei der isopyknischen Dichtegradienten-Zentrifugation mit Cäsiumchlorid aufweisen, ihre Gewebskultur-Infektiosität beim Erhitzen in wäßrigr Suspension für 3 Stunden bei 98°C, jedoch nicht nach 10 Stunden bei 56°C verlieren, gegenüber Äther unempfindlich sind und ein genetisches Material aufweisen, das im histochemischen Anfärbeversuch auf RNA positiv reagiert, wobei diese Teilchen die ATCC-Nr. VR-2015 haben.

2. Diagnostiches Verfahren zum Nachweis eines neuentdeckten ätiologischen Wirkstoffs der NANB-Hepatitis, das die Untersuchung einer biologischen Probe auf Antikörper gegen Virusteilchen mit einem Durchmesser von etwa 27 nm umfaßt, die eine Schwebedichte von 1,36 bei der isopyknischen Dichtegradienten-Zentrifugation mit Cäsiumchlorid aufweisen, ihre Gewebskultur-Infektiosität beim Erhitzen in wäßriger Suspension für 3 Stunden bei 98°C, jedoch nicht nach 10 Stunden bei 56°C verlieren, gegenüber Äther unempfindlich sind und ein genetisches Material aufweisen, das im histochemischen Anfärbeversuch auf RNA positiv reagiert, wobei diese Teilchen die ATCC-Nr. VR-2015 haben.

3. Verfahren nach Anspruch 2, bei dem der Antikörper IgG ist.

4. Verfahren nach Anspruch 2, bei dem der Antikörper IgM ist.

5. Verfahren, das die Kultivierung von ATCC VR-2015 *in vitro* in Zellkultur umfaßt.

6. Verfahren nach Anspruch 5, bei dem die Kultur eine Kultur von diploiden WI-38 oder MRC-5-Zellen ist.

7. Verfahren nach Anspruch 1, bei dem die Probe dadurch auf das genannte Antigen untersucht wird, daß man die Probe mit einem in Wasser unlöslichen Antikörper gegen das genannte Antigen in Berührung bringt, die Probe entfernt, den unlöslichen Antikörper mit einem markierten Antikörper gegen das genannte Antigen in Berührung bringt, den nicht gebundenen markierten Antikörper von dem unlöslichen Antikörper entfernt und entweder die gebundene oder die ungebundene Fraktion auf die Markierung untersucht.

8. Verfahren nach Anspruch 2, bei dem man die Probe dadurch auf den Antikörper untersucht, daß man die Probe mit einem in Wasser unlöslichen spezifischen Bindungspartner für den Antikörper der Testprobe in Berührung bringt, den unlöslichen Antikörper mit einem markierten ATCC VR-2015-Antigen in Berührung bringt, den ungebundenen spezifischen Bindungspartner von dem unlöslichen Antikörper entfernt und entweder die gebundene oder die ungebundene Fraktion auf die Markierung untersucht.

9. Verfahren nach Anspruch 8, bei dem der spezifische Bindungspartner Staphylokokkenprotein A oder ein Antikörper ist.

10. Verfahren nach Anspruch 9, bei dem der Bindungspartner-Antikörper spezifisch für IgG oder IgM ist.

11. Verfahren nach Anspruch 2, bei dem die Probe dadurch auf den genannten Antikörper untersucht wird, daß man die Probe mit einem wasserunlöslichen ATCC VR-2015-Antigen und einem markierten Antikörper gegen dieses Antigen in Berührung bringt, die Probe und den ungebundenen markierten Antikörper von dem unlöslichen Antigen entfernt und entweder die gebundene oder die ungebundene Fraktion auf die Markierung untersucht.

12. Eine Zusammensetzung mit einem Antigen von ATCC VR-2015, die im wesentlichen frei von Primaten-Proteinen ist.

13. Zusammensetzung nach Anspruch 12, bei der das Antigen ein Bestandteil eines intakten ATCC VR-2015-Teilchens ist.

14. Eine sterile Zusammensetzung mit einem ATCC VR-2015-Antigen in einem pharmazeutisch annehmbaren Träger.

15. Zusammensetzung nach Anspruch 14, bei der das Antigen als ein Bestandteil eines biologisch nicht infektiösen ATCC VR-2015-Teilchen vorliegt.

16. Zusammensetzung nach Anspruch 14 oder 15, die trocken ist.

17. Zusammensetzung mit einem Primaten-Immunoglobulin, die im wesentlichen frei von anderen Primaten-Proteinen ist und einen Antikörper zum Binden eines Antigens von ATCC VR-2015 enthält.

18. Zusammensetzung mit einem Nichtprimaten-Antikörper zum Binden eines Antigen von ATCC VR-2015.

19. Zusammensetzung mit einem ATCC VR-2015-Antigen, das an eine nachweisbare Markierung oder einen wasserunlöslichen Träger gebunden ist.

20. Zusammensetzung nach Anspruch 19, bei der die Markierung ein Enzym, ein Radioisotop, eine fluoreszierende Gruppe, ein lumineszierende Gruppe, ein Coenzym, ein Enzymmodulator oder ein stabiles freies Radikal ist.

21. Zusammensetzung mit einem Antikörper gegen ATCC VR-2015-Antigen, der eine nachweisebare Markierung oder einen wasserunlöslichen Träger gebunden ist.

22. Zusammensetzung nach Anspruch 19, bei der der Träger eine Polyolefinoberfläche ist, an die das Antigen adsorbiert ist.

23. Zusammensetzung nach Anspruch 21, bei der der Träger eine Polyolefinoberfläche ist, an die der Antikörper adsorbiert ist.

24. Zusammensetzung mit einem sterilen Gammaglobulin gegen ein ATCC VR-2015-Antigen, wobei das Globulin im wesentlichen frei von dem antihemophilen Faktor und dem Prothrombin-Komplex ist.

25. Zusammensetzung nach Anspruch 24, die trocken ist.

**Revendications**

1. Méthode diagnostique pour la détection d'un agent étiologique nouvellement découvert pour l'hépatite NANB, qui consiste à analyser un échantillon biologique pour détecter l'antigène de particules virales ayant un diamètre d'environ 27 nm, qui montrent une densité de flottaison de 1,36 dans la centrifugation selon un gradient de densité isopycnique avec le chlorure de césium, qui perdent leur infectiosité, dans une culture sur tissu, par chauffage en suspension aqueuse pendant trois heures à 98°C mais non après 10 h à 56°C, étant insensibles à l'éther et ayant un matériel génétique positif vis-à-vis d'un colorant histochimique pour l'ARN, ces particules ayant le N° ATTC VR-2015.

2. Méthode diagnostique pour la détection d'un agent étiologique nouvellement découvert pour l'hépatite NANB, qui consiste à analyser un échantillon biologique pour détecter l'anticorps contre des particules virales ayant un diamètre d'environ 27 nm, qui montrent une densité de flottaison de 1,36 dans la centrifugation selon un gradient de densité isopycnique avec le chlorure de césium, qui perdent leur infectiosité, dans une culture sur tissu, par chauffage en suspension aqueuse pendant trois heures à 98°C mais non après 10 h à 56°C, étant insensibles à l'éther et ayant un matériel génétique positif vis-à-vis d'un colorant histochimique pour l'ARN, ces particules ayant le N° ATTC VR-2015.

3. Méthode suivant la revendication 2, dans laquelle l'anticorps consiste en IgG.

4. Méthode suivant la revendication 2, dans laquelle l'anticorps consiste en IgM.

5. Méthode qui consiste à cultiver ATCC VR-2015 in vitro, en culture cellulaire.

6. Méthode suivant la revendication 5, dans laquelle la culture est conduite dans des cellules diploïdes WI-38 ou MRC-5.

7. Méthode suivant la revendication 1, dans laquelle l'échantillon est analysé en vue de détecter ledit antigène par contact de l'échantillon avec un anticorps insoluble dans l'eau contre ledit antigène, retrait de l'échantillon, contact de l'anticorps insoluble avec un anticorps marqué contre ledit antigène, séparation de l'anticorps marqué non lié de l'anticorps insoluble et titrage du marqueur dans les fractions liées ou non liées.

8. Méthode suivant la revendication 2, dans laquelle l'échantillon est analysé en vue de doser l'anticorps par contact de l'échantillon avec un partenaire de liaison spécifique insoluble dans l'eau pour l'anticorps de l'échantillon analysé, contact de l'anticorps insoluble avec un antigène d'ATCC VR-2015, séparation du partenaire de liaison spécifique non lié de l'anticorps insoluble et titrage du marqueur dans les fractions liées ou non liées.

9. Méthode suivant la revendication 8, dans laquelle le partenaire de liaison spécifique est la protéine A staphylococcique ou un anticorps.

10. Méthode suivant la revendication 9, dans laquelle l'anticorps formant le partenaire de liaison est spécifique d'IgG ou d'IgM.

11. Méthode suivant la revendication 2, dans laquelle l'échantillon est analysé en vue de doser ledit anticorps par contact de l'échantillon avec l'antigène d'ATCC VR-2015 insoluble dans l'eau et un anticorps marqué contre ledit antigène, séparation dudit échantillon et dudit anticorps marqué non lié de l'antigène insoluble et titrage du marqueur dans les fractions liées ou non liées.

12. Composition comprenant un antigène d'ATCC VR-2015 qui est principalement dépourvue de protéines de primate.

13. Composition suivant la revendication 12, dans laquelle l'antigène est un composant d'une particule ATCC VR-2015 intacte.

14. Composition stérile comprenant l'antigène d'ATCC VR-2015 dans un support acceptable du point de vue pharmaceutique.

15. Composition suivant la revendication 14, dans laquelle l'antigène est présent comme composant de particules ATCC VR-2015 biologiquement non infectieuses.

16. Composition suivant la revendication 14 ou 15, sous forme sèche.

17. Composition comprenant une immunoglobuline de primate qui est principalement dépourvue d'autres protéines de primate et qui contient un anticorps pour la fixation d'un antigène d'ATCC VR-2015.

18. Composition comprenant un anticorps non issu de primate pour la fixation d'un antigène d'ATCC VR-2015.

19. Composition comprenant l'antigène D'ATCC VR-2015 lié à un marqueur détectable ou à un support insoluble dans l'eau.

20. Composition suivant la revendication 19, dans laquelle le marqueur est un enzyme, un radio-isotope, un groupe fluorescent, un groupe luminescent, un co-enzyme, un modulateur d'enzyme ou un radical libre stable.

21. Composition comprenant un anticorps contre l'antigène d'ATCC VR-2015 qui est lié à un marqueur détectable ou à un support insoluble dans l'eau.

22. Composition suivant la revendication 19, dans laquelle le support est une polyoléfine à la surface de laquelle l'antigène est adsorbé.

23. Composition suivant la revendication 21, dans laquelle le support est une polyoléfine à la surface de laquelle l'anticorps est adsorbé.

24. Composition comprenant de la gammaglobuline stérile relative à l'antigène d'ATCC VR-2015, cette globuline étant essentiellement dépourvue de facteur antihémophilique et de complexe de prothrombine.

25. Composition suivant la revendication 24, sous une forme sèche.